(19) Europäisches Patentamt
European Patent Office
Office européen des brevets

(11) Veröffentlichungsnummer: **0 114 616**
**B1**

(12) **EUROPÄISCHE PATENTSCHRIFT**

(45) Veröffentlichungstag der Patentschrift: **05.11.86**

(51) Int. Cl.⁴: **D 21 H 3/08,** C 07 C 69/96, C 07 C 103/38, C 07 C 68/02

(21) Anmeldenummer: **84100252.0**

(22) Anmeldetag: **12.01.84**

(54) **Leimungsmittel.**

(30) Priorität: **20.01.83 DE 3301670**

(43) Veröffentlichungstag der Anmeldung: **01.08.84 Patentblatt 84/31**

(45) Bekanntmachung des Hinweises auf die Patenterteilung: **05.11.86 Patentblatt 86/45**

(84) Benannte Vertragsstaaten: **BE CH DE FR GB LI SE**

(56) Entgegenhaltungen:
**DE-A-2 459 165**
**DE-B-2 234 716**
**US-A-3 275 674**

(73) Patentinhaber: **BAYER AG, Konzernverwaltung RP Patentabteilung, D-5090 Leverkusen 1 Bayerwerk (DE)**

(72) Erfinder: **Von Bonin, Wulf, Dr., Mendelssohnstrasse 30, D-5090 Leverkusen 1 (DE)**
Erfinder: **Buschhaus, Hans- Ulrich, Dr., Morgengraben 2, D-5000 Koeln 80 (DE)**
Erfinder: **Beck, Ulrich, Dr., Weiherstrasse 17, D-5303 Bornheim 3 (DE)**
Erfinder: **Bäumgen, Heinz, Buchenweg 10, D-5090 Leverkusen 1 (DE)**

**EP 0 114 616 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Leimungsmittel,insbesondere für Papier auf Basis von Chlorameisensäureestern.

Die Reaktionsfähigkeit von Chlorameisensäureestern ist seit langem bekannt, z.B. die Umsetzung von Chlorameisensäureestern mit OH-Gruppen, gegebenenfalls in Anwesenheit von Säureakzeptoren, die oftmals zu Carbonaten führt. Die Reaktion kann mit primären und sekundären OH-Gruppen, insbesondere aliphatischen OH-Gruppen, wie sie auch in den Kohlehydraten, z.B. in der Stärke oder der Cellulose gegeben sind, erfolgen.

In Anlehnung an diese Kenntnis der Reaktiosweise von Chlorameisenestern ist auch versucht worden, für die Reaktivleimung von Papieren Chlorameisenester der Formel RO-COCl einzusetzen, wobei als R Kohlenwasserstoffreste mit 8 bis 40 Kohlenstoffatomen, wie langkettige aliphatische Reste, Cholesterylreste, u.ä. verwendet wurden. Siehe dazu beispielsweise DE-A-2 234 716.

Diese Produkte haben jedoch gewisse Nachteile, entweder in der Zugänglichkeit, z.B. beim Cholesteryl-Baustein, insbesondere aber in der zu geringen Stabilität wäßriger Formulierungen der reaktiven Chlorameisensäureester einfacher Fettalkohole. Außerdem sind im Gegensatz zu den Fettsäuren höhere Fettalkohole relativ schwer zugängliche Ausgangsprodukte.

Es wurde nun überraschenderweise gefunden, daß sich diese Nachteile insgesamt vermeiden lassen, wenn man als Leimungsmittel nicht Chlorameisensäureester von Kohlenwasserstoff-Alkoholen, (also z.B. von Fettalkoholen), sondern solche von Esteralkoholen und/oder von Carbonsäureamidalkoholen, verwendet.

Die für die neuen Leimungsmittel für Papier eingesetzten Chlorameisensäureester besitzen vorzugsweise folgende idealisierte Struktur.

$(RCOX)_n R' (OCOCl)_m$ (1)

worin

X = ein Sauerstoffatom oder der Rest NH

R = aliphatischer, araliphatischer oder cycloaliphatischer Kohlenwasserstoffrest mit 8 bis 28 Kohlenstoffatomen

R' = Rest eines (n+m)-wertigen Alkohols oder Aminalkoholes mit 2 bis 10 Kohlenstoffatomen, wobei die Wertigkeit sich auf die Gesamtzahl der OH- und $NH_2$-Gruppen bezieht,

n = ganze Zahl von 1 bis 4,

m = ganze Zahl von 1 bis 3.

In Formel (I) steht X vorzugsweise für Sauerstoff, R für einen Kohlenwasserstoffrest mit 11 bis 20 Kohlenstoffatonen, R' enthält vorzugsweise 3 bis 6 C-Atome, n bedeutet vorzugsweise 2 bis 3 und m vorzugsweise 1 bis 2.

Diese Chlorameisensäureester haben gute Leimungswirkung, können in wäßrigem Medium formuliert werden und haben dabei wesentlich bessere Stabilität als die Chlorameisensäureester von einfachen Fettalkoholen und basieren auf leicht zugänglichen Fettsäuren.

Unter den dem Fachmann bekannten Verfahren zur Herstellung von Chlorameisensäureestern ist das Verfahren der Umsetzung von OH-Gruppen enthaltenden Ausgangsmaterialien mit Phosgen bevorzugt zur Herstellung der erfindungsgemäßen Leimungsmittel einsetzbar. Die Herstellung von Chlorameisensäureestern nach derartigen Verfahren entspricht dem Stande der Technik, so daß sie leicht zugänglich sind. Dies gilt ebenfalls für die Herstellung der der Umwandlung in Chlorameisensäurenester zu unterziehenden Fettsäureamid-Alkohole, bzw. insbesondere für die vorzugsweise zum Einsatz gelangenden Fettsäure-esteralkohole, die sich leicht z.B. durch Umsetzung von Fettsäuren, insbesondere technischen Fettsäuregemischen, mit Schmelzpunkten über 30°C mit Polyalkoholen gegebenenfalls im Vakuum oder unter Inertgas bei Temperaturen zwischen 100 und 250°C unter Abtreiben des Reaktionswassers herstellen lassen.

Die Herstellung der Chlorameisensäureester kann wie folgt systematisch dargestellt werden:

A) Herstellung der Chlorameisensäureester (I) mit X = 0:

$$n \cdot R{-}COOH$$

$$\downarrow \; + (OH)_n R'(OH)_m \; - n\,(H_2O)$$

$$(RCOO)_n\, R'(OH)_m \quad (II) \quad \text{(Esteralkohole)}$$

$$\downarrow \; \text{Phosgenierung mit } COCl_2$$

$$(RCOO)_n\, R'(OCOCl)_m \qquad (Ia)$$

B) Herstellung der Chlorameisensäureester (I) mit X = NH:

$$n.R{-}COOH$$

$$\downarrow \; + (H_2N)_n.R'(OH)_m$$

$$(RCONH)_n\, R'(OH)_m \quad (III) \quad \text{(Säureamidalkohole)}$$

$$\downarrow \; \text{Phosgenierung mit } COCl_2$$

$$(RCONH)_n\, R'(OCOCl)_m \qquad (Ib)$$

wobei R, R', m und n die unter Formel (I) angegebene Bedeutung besitzen.

Die Formulierung der Chlorameisensäureester zu Leimungszwecken kann in Form einer Lösung oder Suspension in nicht wäßrigen Medien wie Alkohol, Aceton, Benzin, Toluol, Trichlorethylen leicht schmelzenden oder flüssigen Emulgatoren, Dispergatoren, oder aber vorzugsweise in Form einer wäßrigen Suspension, bzw. Dispersion mit Hilfe von Emulgier- und/oder Dispergierhilfsmitteln etwa wie im Falle der dem Fachmann bekannten Stearyldiketen-Formulierungen erfolgen. In Form dieser wäßrigen Zubereitungen kommt auch der Vorteil der vorteilhaften Lagerstabilität der neuen Leimungsmittel auf Chlorameisensäureesterbasis zum Tragen.

Die erfindungsgemäßen Leimungsmittel können selber noch weitere leimungswirksame Substanzen neben den Chlorameisenestern enthalten, bzw. mit anderen Leimungsmittelzubereitungen gemeinsam oder im Gemisch zum Einsatz gelangen.

Als n + m-wertige Alkohole werden vorzugsweise allein oder im Gemisch miteinander eingesetzt: Ethylenglykol, Propylenglykol, Neopentylglykol, Butandiol, Hexandiol, Glycerin, Trimethylolpropan, Pentaerythrit, Sorbit und andere Zuckeralkohole, insbesondere Glycerin und insbesondere Trimethylolpropan.

Als n + m-wertige Aminalkohole werden vorzugsweise allein oder im Gemisch miteinander eingesetzt: Ethanolamin, Diethanolamin, Triethanolamin, Propanolamin, Dipropanolamin, Tripropanolamin (in n und iso-Form), N-Alkyl-diethanolamine, (N-Dialkylamino)-alkyl-diethanolamine, Anlagerungsprodukte von 1 - 4 Mol Ethylenoxyd und/oder Propylenoxyd an Ethylendiamin.

Als Reste R der Gruppierung (RCO-) mit 8-28-C-Atomen werden vorzugsweise solche verstanden, die sich ableiten lassen von Fettsäuren oder Fettsäuregemischen mit Schmelzpunkten über 30° C, wie Talgfettsäuren, Kokosfettsäuren, Tallfettsäuren, Harzsäuren, Palmitinsäuren, Behensäuren oder insbesondere Stearinsäure und stearinsäurehaltigen Gemischen.

Als Chlorameisensäureester werden erfindungsgemäß solche bevorzugt, die Schmelzpunkte über 10° C, vorzugsweise zwischen 20 und 60° C aufweisen.

Die Chlorameisensäureester können auch in ihrer ungereinigten technischen Herstellungsform eingesetzt werden.

Die Chlorameisensäureester werden gegebenenfalls in anionischer oder eher nichtionischer, insbesondere aber kationischer wäßriger Formulierung mit Wirkstoffgehalten von 1 - 30, vorzugsweise 3 - 15 Gew.-%, eingesetzt.

Die wäßrigen Leimungsmittelzubereitungen stellen bevorzugt Emulsionen oder Dispersionen der erfindungsgemäßen Chlorameisensäureester dar und werden im Anwendungsfalle gegebenenfalls noch weiter auf Wirkstoffkonzentrationen unter 1 Gewichtsprozent verdünnt und mit gegebenenfalls anderen Leimungsmitteln, weiteren Papierhilfsmitteln, wie gegebenenfalls modifizierten Stärken, Füllstoffen, Farbstoffen, Aufhellern, Retentionsmitteln, Fixiermitteln, Naßfestmitteln usw. kombiniert.

Die neuen Leimungsmittel werden gegebenenfalls in der Papieroberfläche, vor allem aber in der Papiermasse eingesetzt.

Als nicht wäßrige Formulierungen kommen im allgemeinen fließfähige, gegebenenfalls aber auch rieselfähige feste Lösungen, bzw. auch Schmelzen der Chlorameisensäureester, bzw. deren Gemische, bzw. Gemische mit anderen Leimungsmitteln oder auch Dispergierhilfsmitteln in Betracht oder auch Lösungen in wassermischbaren oder nicht wassermischbaren Lösungsmitteln oder Lösungsmittelgemischen mit Wirkstoffgehalten von ca. 3 bis ca. 75 Gew.-%. Weiterhin kommen auch Dispersionen in nicht-wäßrigen Lösungsmitteln, bzw. Dispergiermitteln in Betracht, z.B. in Ethanol, Isopropanol, Butanol, Toluol, Trichlorethylen, Aceton, Ethylacetat, Glykolmethylether, Glykolmethyletheracetat, Benzinfraktionen, Erdöldestillate mit Gehalten an Aliphaten, Naphthenen, Aromaten usw., tierische und pflanzliche Öle bzw. Gemische solcher Hilfsmittel.

Die Herstellung wäßriger Formulierungen erfolgt unter Zuhilfenahme von gegebenenfalls Gemischen von in Einzelfällen auch anionischen, bevorzugt jedoch von nicht-ionischen oder insbesondere kationischen niedermolekularen, oligomeren oder polymeren Netz-Emulgier- bzw. Dispergierhilfsmitteln bei Temperaturen vorzugsweise kurz oberhalb des Schmelzpunktes der Chlorameisensäureester mittels üblicher dem stand der Technik entsprechender Emulgiergeräte, wie z.B. Rührer oder Rotor-Stator-, Düsen- oder Ultraschallgeräte, mit anschließend möglichst schnellem Abkühlen unter den Kristallitschmelzpunkt, bzw. stärkeres Abkühlen, z.B. bis auf Temperaturen um 4° C.

Als Hilfsmittel zur Herstellung der wäßrigen Dispersionen kommen beispielsweise dem Fachmann geläufige Stoffgruppen in Betracht, mit netzenden, emulgierenden, dispergierenden oder stabilisierenden Eigenschaften, wobei deren Mengen bezogen auf Chlorameisensäureester zwar möglichst gering gehalten werden sollten, aber dann, wenn sie selbst leimenden Charakter haben, auch die Menge des in der Formulierung enthaltenden Chlorameisensäureesters um das Mehrfache übersteigen kann.

Im Falle, daß die zugesetzten Hilfsmittel keinen leimenden oder einen die Leimung prinzipiell beeinträchtigenden Charakter haben, sollte ihre Einsatzmenge bezogen auf Chlorameisensäureester unter 80 Gew.-%, vorzugsweise zwischen 2 bis 50 Gew.-% liegen.

Im Falle, daß die als Dispergierhilfsmittel verwendeten Substanzen evtl. selbst leimenden Charakter haben, bzw. sogar selbst Leimungsmittel, bzw. Leimungsmittelformulierungen darstellen, können die erfindungsgemäß einzusetzenden Chlorameisensäureester selbstverständlich auch in wesentlich geringeren Anteilen im Gesamtwirkstoff mit Leimungswirkung in der wäßrigen Formulierung enthalten sein, d.h. als Zusatz zu den sonstigen leimenden Komponenten angewendet werden.

Als Dispergierhilfsmittel mit Leimungsmittelcharakter können hier als solche oder in Form ihrer Zubereitungen in wäßriger Formulierung verstanden werden: quarternierte oder nicht quarternierte basische Polymere, Oligomere, Fettsäureamide, Stärken, Kollophonium- oder Kohlenwasserstoffharze oder auch prinzipiell nicht mit basischen Gruppen versehene aber in kationischer bzw. nicht-ionischer wäßriger Zubereitung vorliegende Leimungsmittel auf Tallharzbasis, auf Basis von Fettalkyl-diketen-präparaten, Kohlenwasserstoffharzen, oder Kollophoniumharzen, bzw. Wollfetten. Als Dispergierhilfsmittel ohne eigene Leimungswirkung kommen in Betracht beispielsweise kationische Poly- oder Oligoaddukte bzw. -kondensate, wie sie als sogenannte Leimungsverstärker, als Retentionsmittel oder Naßfestmittel in der Papiertechnologie bekannt sind, Stärke, kationische Stärken, Cellulosen, Chitinpräparate, Harzsäureabkömmlinge oder sonstige kationisch modifizierte Poly- oder Oligosaccharide zumeist pflanzlicher Herkunft, gegebenenfalls in Kombination mit ionischen Tensiden, z.B. Naphthalin- oder Ligninsulfonate, Alkylsulfate, Fettsäuresalze, guartäre Fettammoniumsalze oder insbesondere nicht ionischen Tensiden des Standes der Technik, seien es guartäre, aliphatische oder araliphatische Ammoniumverbindungen, alkoxylierte Harzsäuren, Fettsäuren, Fettalkohole, Isononylphenole, Lecithine, Proteine oder Fette, bzw. Kohlenhydrate usw.

Die neuen Leimungsmittel werden in Gestalt ihrer Formulierungen zur Ausrüstung der Papiere in der Oberfläche oder bevorzugt in der Masse zugesetzt.

Die wäßrigen Formulierungen haben Wirkstoffgehalte an Leimungsmittel von 1 bis 30 %, vorzugsweise 3 bis 15 Gew.-%. Sie können vor dem Einsatz in der Papiermasse oder Leimungsflotte noch weiter, z.B. auf Wirkstoffgehalte von 0,1 bis 1 % verdünnt werden.

Die neuen Leimungsmittel haben den Vorteil, in Form ihrer wäßrigen Zubereitungen wesentlich stabiler zu sein als die Chlorameisensäureester einfacher Fettalkohole, andererseits aber über Reaktivgruppen zu verfügen, die offenbar noch so gut mit dem Papierstoff reagieren können, daß unmittelbar nach der Trocknung des Papiers auch bei relativ niedrigen, etwa um 80°C liegenden Temperaturen, bei denen z.B. Epichlorhydrin-Umsetzungsprodukte oder Fettalkyl-diketen-Leimungsmittel noch nicht oder nur langsam ansprechen, schon

ihre volle Leimungswirkung zu entfalten, also eine Sofortleimung zu bewirken, die unmittelbar hinter der Papiermaschine beurteilt werden kann.

Es sind keine Zusätze von Alaun oder sonstigen Papierhilfsmitteln erforderlich, obgleich ein Zusatz solcher Hilfsmittel, z.B. auf Basis kationischer Stärke, quaternierten Polyaminen, quaternierten Polyamidaminen, quaternierten basischen Formaldehydharzen, Methylcellulose, Carboxynethylcellulose, Ligninsulfonsäuren, Stärken und Polysacchariden verschiedenster Genese, Xanthan, Pullulan, Chitosan, Polymerisaten oder Copolymerisaten von (Meth)-Acrylsäure, Malein-, Fumar-, Itaconsäure oder sonstigen Polymeren und Copolymeren mit gegebenenfalls in Salzform vorliegenden Carboxyl- oder Sulfonsäuregruppen, Kollagen, Gelatine, Alginaten und Karagenaten, durchaus in Betracht zu ziehen und gegebenenfalls möglich ist. Ebenso ist die Kombination mit Leichtfüllstoffen, Mikrokapseln, löslichen und unlöslichen Farbstoffen oder sonstigen Papier-Zusatzstoffen möglich.

Die Wirksamkeit der neuen Leimungsmittel wird durch Weißtöner nicht verschlechtert.

Die Leimungsmittel sind allein oder in Kombination mit anderen Leimungsmitteln besonders gut geeignet zur Masseleimung von Papier, können gegebenenfalls aber auch zur Oberflächenleimung eingesetzt werden. Sie können nicht nur bei kreidehaltigen oder eventuell auch kaolinhaltigen Papieren verwendet werden, sondern auch bei solchen, die keinen oder einen andersartigen Füllstoff enthalten, wie z.B. Talkum oder Gips. Ebenso sind sie zur Leimung von cellulosischen Materialien wie Pappe, synthetischen Pulpen, Textilmaterial, Leder, Karton oder Holzspanplatten bzw. Dämmplatten geeignet.

Im folgenden soll die Erfindung beispielhaft erläutert werden; die angegebenen Teile und Prozente beziehen sich auf das Gewicht, sofern nichts anderes vermerkt ist.

**Herstellungsbeispiele**

Die Herstellung der Chlorameisenester erfolgt, indem 1 Mol n + m-wertiger Alkohol und n Mol technische Fettsäure unter Stickstoff und Abtreiben des Kondensationswassers 10 h bei 170°C kondensiert werden. Dabei entsteht der Esteralkohol mit einer Säurezahl unter 12. Dieser wird in toluolischer Lösung phosgeniert und durch Abdestillieren des Toluols als Chlorameisenester isoliert.

Die Chlorameisensäureester, die hier zum Einsatz kommen, schmelzen alle oberhalb 25°C und unter 50°C.

Als Vergleichssubstanz dient aus Stearylalkohol durch analoge Phosgenierung hergestelltes Material.

Es werden folgende Bezeichnungen benutzt:

Vergleichswirkstoff Basis: Stearylalkohol

Wirkstoff A Basiś: 1 Mol Glycerin, 2 Mol techn. Kokosfettsäure

Wirkstoff B Basis: 1 Mol Trimethylolpropan, 2 Mol techn. Stearinsäure.

Als Dispergierhilfsmittel wurden verwendet:

Hilfsmittel A Copolymerisat aus 30 % N-(3-dimethylamino-propyl)-maleinimid, 10 % Styrol und 60 % Butylacrylat, mit Epichlorhydrin guarterniert (Beispiel 17 der DE-A- 30 46 981)

Hilfsmittel B handelsübliche kationische Stärke

Hilfsmittel C Dimethyl-oleyl-Benzyl-ammoniumchlorid in Wasser

Hilfsmittel D Polyvinylalkohol

Hilfsmittel E Amid aus 2 Mol Ölsäure und 1 Mol Triethylentetramin, umgesetzt mit ca. 3 Mol Epichlorhydrin.

Als Emulgiergerät wird eine Rotor-Stator-Emulgieranlage verwendet. (Megatron, Fa. Kinematica GmbH).

Die Herstellung der wäßrigen Leimungsmittelzubereitungen erfolgte so, daß zunächst das Wasser im Gemisch mit den Eilfsmittel bei 50°C in der Mischanlage im Umlauf gebracht wurde. Dann wurde bei 90 % der Höchstdrehzahl (9000 Upmin) der Chlorameisenester in Form der auf 50°C vorerhitzten Schmelze hinzuemulgiert und die Mischung bei 50°C 1 Minute im Umlauf gehalten, das Umlaufvolumen betrug 200 ml. Dann wurde im Laufe von 3 Minuten auf 18°C abgekühlt und die entstandene stabile Emulsion als Leimungsmittel eingesetzt; die Teilchendurchmesser der Emulsion liegen zwischen etwa 1,5 und 100 µm.

Ein wesentlicher Vorteil der Reaktiv-Leimungsmittel ergibt sich daraus, daß die Leimungsmittel auf alaunfreien und kreidehaltigen Papieren, auf denen Harzleim nur wenig wirksam ist, ihre gute Wirksamkeit entfalten. Die erfindungsgemäßen Leimungsmittel werden daher auch beispielhaft auf alaunfreiem, kreidehaltigem Papier geprüft.

In 200 ml Leitungswasser werden 5 g einer Mischung aus 50 g Fichten-Sulfitzellstoff, 50 g Buchen-Sulfatzellstoff und 25 g Kreide aufgeschlämmt. Dann werden x % des Leimungsmittels (Wirkstoff bezogen auf Zellstoff plus Füllstoff) hinzugerührt. Dann wird ohne Zusatz eines Fixiermittels mit Wasser auf ca. 1 Liter aufgefüllt und auf einem Blattbildner das Papierblatt hergestellt. Dieses wird abgesaugt, abgepreßt und auf einem Trockenzylinder bei 110°C 5 Min. getrocknet. Aus dem Blatt werden für die Tintenschwimmprobe Streifen (2 cm x 6 cm) geschnitten und ausgeprüft.

Als Beurteilungskriterium für die Leimungsmittel wird die sogenannte Tinten-Schwimmprobe benutzt: Man legt mit dem zu testenden Mittel ausgerüsteten Papierstreifen auf die Oberfläche einer mit Normtinte gemäß DIN 53 126 gefüllten Schale und prüft die Zeit, die vergeht, bis die Tinte auf die dem Betrachter zugekehrte Seite des aufgelegten Papiers durchschlägt.

Dieser Test liefert bei standardisierter Durchführung eine sehr gute Beurteilungsmöglichkeit für verschiedene Leimungsmittel.

Eine andere Beurteilungsmöglichkeit liegt in der Bestimmung der Wasseraufnahme der zu prüfenden Papiere nach Cobb gemäß DIN 53 132 (Nov. 1965) nach 60 Sek. Einwirkungszeit: Je geringer die gemessene Wasseraufnahme (Cobb-Wert), desto besser die Leimungswirkung des Leimungsmittels.
Im folgenden sind die erläuternden Beispiele tabellarisch mit den Beurteilungen zusammengefaßt worden:

| Beispiel-Nr. | 1 | 2 | 3 | 4 | 5 | 6 | 7 | 8 | 9 | 10 | 11 |
|---|---|---|---|---|---|---|---|---|---|---|---|
| Wirkstofftyp; % | Vergleich; 6 | → | → | → | B;6 | → | → | → | A;6 | A;12 | → |
| Hilfsmitteltyp; % | A;2 | → | → | → | A;2 | → | → | → | B;1 | A;2 | → |
| Hilfsmitteltyp; % | | | | | | | | | C;1 | | |
| Hilfsmitteltyp; % | | | | | | | | | | D;0,5 | → |
| Hilfsmitteltyp; % | | | | | | | | | E;1 | | |
| Wassermenge; % | 92 | → | → | → | 92 | → | → | → | 91 | 85,5 | → |
| Standzeit 20° C (h) | 3 | 60 | | | 3 | 240 | | | | 3 | |
| Standzeit 8° C (Tage) | | | 3 | 10 | | | 10 | 100 | 10 | | 100 |
| Einsatzmenge (% Wirkstoff) | 0,3 | 0,3 | 0,3 | 0,3 | 0,3 | 0,3 | 0,3 | 0,3 | 0,3 | 0,3 | 0,3 |
| Tintenzeit (ca. Sek.) | 800 | * | 200 | <10 | 850 | 320 | 840 | 705 | 415 | 930 | 825 |
| Cobb-Wert | 21 | * | 86 | 100 | 25 | 55 | 25 | 27 | 32 | 21 | 25 |

* Dispersion ist gebrochen und hat sich verfestigt

Die tabellarisch aufgeführten Versuche zeigen, daß die neuen Chlorameisensäureester-Leimungsmittel eine gegenüber den einfachen Chlorameisensäureestern auf Fettalkoholbasis wesentlich verbesserte Stabilität in wäßrigem Milieu besitzen, und daß sehr unterschiedliche Formulierungsmöglichkeiten bestehen.

**Beispiel 12**

70 Gew.-Teile Wirkstoff B mit einem Schmelzintervall von 25 - 33°C werden mit 30 Gew.-Teilen eines Anlagerungsproduktes von 10 Mol Ethylenoxid an Isononylphenol, das an der OH-Gruppe cyanethyliert ist, versetzt. Es bildet sich eine gut fließfähige Lösung. Diese Lösung wird als nicht wäßrige Leimungsmittelformulierung verwendet, indem man sie dem kreidehaltigen Papierstoff unter Rühren in einer Einsatzmenge von 0,3 % (in Form des enthaltenen Wirkstoffes) zusetzt, wobei sich das Leimungsmittel gut verteilt. Das gemäß der für Beispiel 1 - 11 geltenden Arbeitsweise hergestellte und geprüfte Papiermuster hat einen Cobb-Wert von 29,5. Der Vorteil derartiger Zubereitungen liegt darin, daß die Formulierung naturgemäß gut als Flüssigkeit dosierbar und wegen der Abwesenheit von Wasser beliebig lange lagerfähig ist.

**Beispiel 13**

Das ungeleimte kreidehaltige Versuchspapier wird eine Sekunde lang in eine 0,15 %ige toluolische Lösung des Wirkstofftyps B getaucht, dann zwischen Filterpapierscheiben mit 1 981.$10^2$ Pa abgequetscht und bei 100° C in einer Wasserdampf gesättigten Atmosphäre 3 Min. lang ausgeheizt. Bei dem so oberflächlich behandelten Papier mißt man nach dem Konditionieren bei Raumtemperatur eine Tintenschwimmzeit von 650 s.

**Patentansprüche**

1. Leimungsmittel, insbesondere für Papier, dadurch gekennzeichnet, daß sie Chlorameisensäureester von Esteralkoholen und/oder von Carbonsäureanidalkoholen enthalten.

2. Leimungsmittel gemäß Anspruch 1, dadurch gekennzeichnet, daß sie Chlorameisensäureester folgender idealisierter Struktur enthalten:

$(RCOX)_n R''(OCOCl)_m$ (I)

worin

X = ein Sauerstoffatom oder der Rest HH

R = aliphatischer, araliphatischer oder cycloaliphatischer Kohlenwaserstoffrest mit 8 bis 28 Kohlenstoffatomen

R' = Rest eines (n + m)wertigen Alkohols oder Aminalkohols mit 2 bis 10 Kohlenstoffatomen, wobei die Wertigkeit sich auf die Gesamtzahl der OH- und $NH_2$-Gruppe n bezieht,

n = ganze Zahlen von 1 bis 4

m = ganze Zahlen von 1 bis 3

3. Leimungsmittel gemäß Anspruch 2, dadurch gekennzeichnet, daß in der Formel I

X = ein Sauerstoffatom,

R = ein Kohlenwasserstoffrest mit 11 bis 20 Kohlenstoffatomen,

R' = Rest eines (n+m)-wertigen Alkohols mit 3 bis 6 Kohlenstoffatomen,

n = 2 oder 3 und

m = 1 oder 2.

4. Leimungsmittel nach Anspruch 1 bis 3, dadurch gekennzeichnet, daß die darin enthaltenen Chlorameisensäureester Schmelzpunkte zwischen 10 und 60° C aufweisen.

5. Leimungsmittel nach Anspruch 2 bis 4, dadurch gekennzeichnet, daß sich die Gruppierung RCO der Formel I von Fettsäuren oder Fettsäuregemischen mit Schmelzpunkten über 30° C und sich R' von Trimethylolpropan ableitet.

6. Leimungsnittel nach Anspruch 2 bis 5, dadurch gekennzeichnet, daß m = 1.

7. Leimungsmittel nach Anspruch 1 - 6, dadurch gekennzeichnet, daß sie Chlorameisensäureester in Form ihrer technischen Rohprodukte enthalten.

8. Leimungsnittel nach Anspruch 1 - 7, dadurch gekennzeichnet, daß sie Chlorameisensäureester in Kombination bzw. Abmischung mit weiteren Komponenten mit Leimungsmittelfunktion enthalten.

9. Leimungsmittel nach Anspruch 1 - 8, dadurch gekennzeichnet, daß die Chlorameisensäureester in Form wäßriger Formulierungen vorliegen.

10. Verfahren zur Herstellung von Leimungsmitteln gemäß Anspruch 2, dadurch gekennzeichnet, daß Esteralkohole und/oder Carbonsäureamidalkohole der Formel

$(R\text{-}COX)_n R''(OH)_m$

worin R, X, R', n und m die in Anspruch 2 angegebene Bedeutung besitzen, zu Chlorameisensäureestern der

Formel (I) phosgeniert und die so erhaltenen Chlorameisensäureester, gegebenenfalls unter Zusatz weiterer Komponenten, zu wäßrigen oder nichtwäßrigen Leimungsmittelpräparaten formuliert werden.

**Claims**

1.Sizing agents, in particular for paper, characterised in that they contain chloroformic acid esters of ester alcohols and/or of carboxylic acid amide alcohols.

2. Sizing agents according to Claim 1, characterised in that they contain chloroformic acid esters of the following idealised structure:

$(RCOX)_n R'(OCOCl)_m$

wherein

X = an oxygen atom or the radical NH

R = an aliphatic, araliphatic or cycloaliphatic hydrocarbon radical with 8 to 28 carbon atoms,

R' = the radical of an (n+m)functional alcohol or amino alcohol with 2 to 10 carbon atoms, the functionality referring to the total number of the OH and $NH_2$ groups,

n = integers from 1 to 4, and

m = integers from 1 to 3.

3. Sizing agents according to Claim 2, characterised in that in the formula I

X = an oxygen atom,

R = a hydrocarbon radical with 11 to 20 carbon atoms,

R' = the radical of an (n+m)-functional alcohol with 3 to 6 carbon atoms,

n = 2 or 3 and

m = 1 or 2.

4. Sizing agents according to Claim 1 to 3, characterised in that the chloroformic acid esters contained therein have melting points between 10 and 60° C.

5. Sizing agents according to Claim 2 to 4, characterised in that the grouping RCO of the formula I is derived from fatty acids or fatty acid mixtures having melting points above 30°C and R' is derived from trimethylolpropane.

6. Sizing agents according to Claim 2 to 5, characterised in that m = 1.

7. Sizing agents according to Claim 1 - 6, characterised in that they contain chloroformic acid esters in the form of their technical crude products.

8. Sizing agents according to Claim 1 - 7, characterised in that they contain chloroformic acid esters in combination or mixed with further components having a sizing agent function.

9. Sizing agents according to Claim 1 - 8, characterised in that the chloroformic acid esters are in the form of aqueous formulations.

10. Process for the preparation of sizing agents according to Claim 2, characterised in that ester alcohols and/or carboxylic acid amide alcohols of the formula

$(R\text{-}COX)_n R'(OH)_m$

wherein

R, X, R', n and m have the meaning given in Claim 2, are phosgenated to form chloroformic acid esters of the formula I) and the chloroformic acid esters thus obtained are formulated into aqueous or non-aqueous sizing agent preparations, optionally with the addition of further components.

**Revendications**

1. Agents d'encollage, en particulier pour papier, caractérisés en ce qu'ils contiennent des esters chloroformiques d'esters-alcools et/ou de carboxamides-alcools.

2. Agents d'encollage selon la revendication 1, caractérisés en ce qu'ils contiennent des esters chloroformiques de structure idéalisée suivante:

$(RCOX)_n R' (OCOCl)_m$ (I)

dans laquelle

X = un atome d'oxygène ou le radical NH

R = radical hydrocarboné aliphatique, araliphatique ou cycloaliphatique ayant 8 à 28 atomes de carbone

R'= radical d'un alcool ou aminoalcool de valence (n+m) ayant 2 à 10 atomes de carbone, la valence se rapportant au nombre total des groupes OH et $NH_2$,

n = nombres entiers de 1 à 4

m = nombres entiers de 1 à 3.

3. Agents d'encollage selon la revendication 2, caractérisés en ce que dans la formule I

X = un atome d'oxygène

R = un radical hydrocarboné ayant 11 à 20 atomes de carbone

R'= un radical d'un alcool de valence (n+m) ayant 3 à 6 atomes de carbone

n = 2 ou 3 et
m = 1 ou 2.

4. Agents d'encollage selon les revendications 1 à 3, caractérisés en ce que les esters chloroformiques qui y sont contenus présentent des points de fusion entre 10 et 60°C.

5. Agents d'encollage selon les revendications 2 à 4, caractérisés en ce que le qroupement RCO de la formule I dérive d'acides gras ou de mélanges d'acides gras ayant des points de fusion supérieurs à 30°C et en ce que R' dérive du triméthylolpropane.

6. Agents d'encollage selon les revendications 2 à 5, caractérisés en ce que m = 1.

7. Agents d'encollage selon les revendications 1 à 6, caractérisés en ce qu'ils contiennent les esters chloroformiques sous forme de leurs produits bruts techniques.

8. Agents d'encollage selon les revendications 1 à 7, caractérisés en ce qu'ils contiennent les esters chloroformiques en combinaison ou en coupage avec d'autres composants exerçant la fonction d'agent d'encollage.

9. Agents d'encollage selon les revendications 1 à 8, caractérisés en ce que les esters chloroformiques se présentent sous la forme de formulations aqueuses.

10. Procédé de fabrication d'agents d'encollage selon la revendication 2, caractérisé en ce qu'on phosgène des esters-alcools et/ou des carboxamides-alcools de formule:

$(R\text{-}COX)_n R' (OH)_m$

dans laquelle R, X, R', n et m possèdent la signification indiquée à la revendication 2, pour obtenir les esters chloroformiques de formule (I) et en ce qu'on formule les esters chloroformiques ainsi obtenus, éventuellement avec addition d'autres composants, en des préparations d'agents d'encollage, aqueuses ou non aqueuses.